# EUROPÄISCHE PATENTSCHRIFT

(11) **EP 1 563 288 B1**
(45) Veröffentlichungstag und Bekanntmachung des Hinweises auf die Patenterteilung: **08.09.2010**
(21) Anmeldenummer: 03785515.2
(22) Anmeldetag: 14.11.2003
(51) Int. Cl.: G01N 27/28, G01N 33/487

(54) **MESSGERÄT ZUR BESTIMMUNG EINES ANALYTEN IN EINER FLÜSSIGKEITSPROBE UNTER VERWENDUNG VON POLYMERELEKTRONISCHEN GERÄTEKOMPONENTEN**
MEASURING APPARATUS USED FOR DETERMINING AN ANALYTE IN A LIQUID SAMPLE, COMPRISING POLYMER ELECTRONIC COMPONENTS
APPAREIL DE MESURE POUR DETERMINER UN ANALYTE DANS UN ECHANTILLON LIQUIDE COMPORTANT DES COMPOSANTS ELECTRONIQUES POLYMERES

(30) Priorität: 14.11.2002 DE 10253154
(43) Veröffentlichungstag der Anmeldung: 17.08.2005
(73) Patentinhaber: PolyIC GmbH & Co. KG, 90763 Fürth (DE); Roche Diagnostics GmbH, 68305 Mannheim (DE); F.HOFFMANN-LA ROCHE AG, 4070 Basel (CH)
(72) Erfinder: CLEMENS, Wolfgang, 90617 Puschendorf (DE); GERLT, Axel, 90766 Fürth (DE); RÖSICKE, Bernd, 68305 Mannheim (DE)
(74) Vertreter: Zinsinger, Norbert
(86) Internationale Anmeldenummer: PCT/DE2003/003784
(87) Internationale Veröffentlichungsnummer: WO 2004/044571

(56) Entgegenhaltungen:
- WO-A-01/73109
- US-A- 5 395 504
- US-A- 5 502 396
- US-A- 5 580 794
- US-A- 5 630 986
- US-A1- 2002 130 042
- "POLYTRONIC: CHIPS VON DER ROLLE" FRAUNHOFER MAGAZIN, Nr. 4, 2001, Seiten 8-12, XP002277252 MÜNCHEN
- HARSANYI G ET AL: "Polytronics for biotronies: unique possibilities of polymers in biosensors and BioMEMS ?" IEEE POLYTRONIC 2002 CONFERENCE, 23. Juni 2002 (2002-06-23), Seiten 211-215, XP010594260

## Beschreibung

Die Erfindung betrifft ein Messgerät zur Bestimmung eines Analyten in einer Flüssigkeitsprobe, mit folgenden Gerätekomponenten: ein Testelement mit einem Testfeld zur Aufbringung der Flüssigkeitsprobe, wobei das Testelement durch den zu bestimmenden Analyten eine detektierbare Veränderung erfährt, ein Detektor, der die Veränderung detektiert und in Abhängigkeit davon ein elektrisches Detektorsignal erzeugt, eine dem Detektor nachgeordnete Auswerteschaltung, die das Detektorsignal zu einem Messergebnis auswertet, eine an der Auswerteschaltung angeschlossene Anzeigevorrichtung zur Anzeige des Messergebnisses und eine Stromversorgungseinrichtung zur Stromversorgung der elektrischen Gerätekomponenten.

Ein derartiges, aus der US 6 300 141 bekanntes Messgerät ist auf einem Träger, beispielsweise in der Form und Größe einer Kreditkarte, ausgebildet und besteht aus einem unteren Trägerteil und einem farbigen oder durchsichtigen Abdeckteil, die beide aus Kunststoff sein können und miteinander verbunden, beispielsweise verklebt, sind. Zwischen dem unteren Trägerteil und dem Abdeckteil sind ein von dem Testelement mit dem Detektor gebildeter elektrochemischer Biosensor, die aus einem Mikroprozessor mit Speicher bestehende Auswerteschaltung, die z. B. aus einem Flüssigkristalldisplay bestehende Anzeigevorrichtung, ein Bedienelement sowie die aus einer Solarzelle oder Batterie bestehende Stromversorgungseinrichtung angeordnet. Das in dem Abdeckteil enthaltene Testfeld zur Aufbringung der Flüssigkeitsprobe kommuniziert über einen Flüssigkeitspfad mit dem Biosensor, wobei der Flüssigkeitspfad und der Biosensor entweder in dem unteren Trägerteil oder auf einem separaten Chip ausgebildet sind, der an dem Träger ansteckbar ist. Zur Bestimmung von mehreren Analyten in der Flüssigkeitsprobe können mehrere Biosensoren vorgesehen sein, die dann über getrennte Flüssigkeitspfade mit dem einen Testfeld zur Aufbringung der Flüssigkeitsprobe verbunden sind.

Ein vergleichbares Messgerät mit elektrooptischem Biosensor ist aus der US 5 580 794 bekannt. Dort sind u. a auch Möglichkeiten angegeben, das Messgerät während oder nach seiner Herstellung zu kalibrieren.

Die bekannten kartenförmigen Messgeräte ermöglichen auf einfache Weise analytische Diagnosen in den Bereichen Gesundheit, Lebensmittel und Umwelt, wobei das Messergebnis unmittelbar von der Karte ablesbar ist. Darüber hinaus kann das Messergebnis auch auf der Karte gespeichert und durch ein externes Lesegerät ausgelesen werden. Bei dem kartenförmigen Messgerät kann es sich um einen Wegwerfartikel handeln. Da der Flüssigkeitspfad und gegebenenfalls der Biosensor nach einer erfolgten Messung nicht mehr zu gebrauchen sind, müsste dann, wenn der Flüssigkeitspfad und der Biosensor auf der Karte selbst ausgebildet sind, das ganze kartenförmige Messgerät weggeworfen werden. Dies ist jedoch aus Kostengründen nur dann praktizierbar, wenn die Messungen vergleichsweise selten, also nur wenige Male im Jahr, stattfinden.

Der Erfindung liegt daher die Aufgabe zugrunde, eine.einfache und bequem handhabbare und dabei kostengünstige Bestimmung eines Analyten in einer Flüssigkeitsprobe zu ermöglichen.

Wie aus dem Fraunhofer Magazin 4, 2001, Seiten 8 bis 13 bekannt ist, eröffnen seit einiger Zeit leitende oder halbleitende Kunststoffe einen Weg zur billigen Massenfertigung von elektronischen Bauelementen und Schaltungen. Beispiele dafür sind Folienbatterien, organische Solarzellen, Displays aus organischen Leuchtdioden (OLEDs) und integrierte Schaltungen aus (halb)leitenden organischen Materialien, wie z. B. Polymeren (Integrated Plasic Circuits = IPCs). Für diese neue Technik werden Begriffe wie organische Elektronik, Polymerelektronik, Polytronik, Electronic Plastics oder Conductive Polymers oft synonym verwendet.

Gemäß der Erfindung wird die oben genannte Aufgabe dadurch gelöst, dass bei dem Messgerät der eingangs angegebenen Art die elektrischen Gerätekomponenten zumindest teilweise auf der Basis von Polymerelektronik ausgebildet sind. Vorzugsweise sind alle elektrischen Gerätekomponenten auf der Basis von Polymerelektronik ausgebildet. Das erfindungsgemäße Messgerät ist dadurch besonders preiswert herstellbar, so dass es beispielweise als Einwegartikel auch für häufige, z. B. tägliche, Messungen infrage kommt. So können die in Polymerelektronik realisierten Gerätekomponenten beispielsweise in Drucktechnik, z. B. unter Verwendung von Polymeren in Lösung (sog. elektronische Tinte), auf einem flachen Träger (Karte, Folie o. ä.) aufgebracht werden. Da Druck- und Laminiertechniken auch für die Herstellung von elektrochemischen Sensoren (Glucosesensoren) bekannt sind, lässt sich so das komplette Messgerät in einem einzigen drucktechnischen Prozess auf dem Träger aufbringen. Dabei können sämtliche Gerätekomponenten des Messgeräts auf einem Träger integriert werden oder es kann vorgesehen werden, dass das Testfeld und das Testelement oder das Testfeld, das Testelement und der Detektor in einer ersten Geräteeinheit ausgebildet sind, die über eine Schnittstelle an einer die übrigen Gerätekomponenten enthaltenden weiteren Geräteeinheit ankoppelbar ist. Die Gerätekomponenten der ersten Geräteeinheit sind dann beispielsweise auf einem separaten und damit auswechselbaren Träger ausgebildet, der an dem Träger mit den übrigen Gerätekomponenten.befestigbar, z. B. ansteckbar, ist.

Die Gerätekomponenten des erfindungsgemäßen Messgerätes umfassen vorzugsweise weiterhin eine Steuereinrichtung, die den Messvorgang für die Bestimmung des Analyten steuert. Zum Aktivieren des Messvorganges kann die Steuereinrichtung Mittel aufweisen, die auf die Betätigung eines Bedienelementes am Messgerät, z. B. eines Bedienknopfes, auf das Entfernen einer Abdeckung am Messgerät oder auf das Aufbringen der Flüssigkeitsprobe auf das Testfeld ansprechen. Bei der Abdeckung kann es sich beispielsweise um eine Folie handeln, die das Messgerät ganz oder bereichsweise, z. B. im Bereich des Testfeldes und/oder eines elektrooptischen Elements (z. B. Solarzelle), abdeckt und deren Entfernen elektrisch, z. B. durch Herstellen oder Unterbrechen eines Kontakts, oder optisch detektiert wird. Die Abdeckung, welche im Weiteren das Messgerät zumindest im Bereich des Testfeldes gegenüber Umgebungseinflüssen wie Wasserdampf und/oder Sauerstoff abdichten kann, kann auch aus einer Verpackung für das komplette Messgerät bestehen. Um das Testfeld und die Polymerelektronik gegen Licht zu schützen, ist die Abdeckung darüber hinaus vorzugsweise lichtundurchlässig. Ist das Entfernen der Abdeckung, z. B. Folie oder Beschichtung, vor dem bestimmungsgemäßen Gebrauch des Messgeräts nicht vorgesehen, so ist die Abdeckung zumindest bereichsweise, insbesondere im Bereich der Anzeigevorrichtung und einer als Stromversorgungseinrichtung dienenden Solarzelle, transparent ausgebildet.

Die Steuereinrichtung weist ferner in vorteilhafter Weise Mittel zum Verhindern weiterer Messvorgänge mit ein und demselben Testelement auf, so dass eine Wieder- bzw. Mehrfachbenutzung eines bereits benutzten Testelements ausgeschlossen wird. Weist das Messgerät nur ein einziges Testelement auf, so betrifft der Wiederverwendungsschutz das komplette Messgerät. Weist das Messgerät, wie unten noch näher erläutert wird, mehrere Testelemente auf, so betrifft der Wiederverwendungsschutz zunächst jedes einzelne Testelement und erst dann, wenn alle Testelemente benutzt worden sind, auch das Messgerät selbst.

In diesem Zusammenhang weist das Messgerät vorzugsweise Anzeigemittel auf, die durch die Steuereinrichtung zur Anzeige der Unbrauchbarkeit des Testelements bzw. des Messgerätes ansteuerbar sind. Die Unbrauchbarkeit ist nach einmaliger Benutzung des Testelements am Ende des betreffenden Messvorganges gegeben. Darüber hinaus kann die Unbrauchbarkeit auch bei noch nicht benutztem Testelement durch Detektieren und Auswerten vorgegebener Parameter des Testelements, beispielsweise seiner elektrische Leitfähigkeit, festgestellt werden, Die Anzeige der Unbrauchbarkeit kann über die Anzeigevorrichtung des Messgeräts erfolgen. Um dabei die Unbrauchbarkeit unabhängig von der Stromversorgung dauerhaft anzeigen zu können, kann die Anzeige bistabil, d. h. zwischen einem anzeigenden Zustand und einem nichtanzeigenden Zustand umschaltbar, erfolgen, wobei nur für die Umschaltung Energie benötigt wird. Alternativ kann die Anzeige beispielsweise dadurch erfolgen, dass an einer Stelle des Messgeräts durch elektrochemische oder -thermische Ansteuerung eine chemische Reaktion ausgelöst wird, die einen Farbumschlag zur Folge hat.

Die Stromversorgungseinrichtung kann aus einer Solarzelle oder Batterie, vorzugsweise auch in Polymerelektronik, bestehen. Im Falle der Batterie kann vorgesehen werden, dass der Elektrolyt erst durch eine manuelle Betätigung an dem Messgerät, z. B. Pressen eines Bedienfeldes, Aufreißen der Verpackung u ä., in Kontakt mit den Elektroden der Batterie gebracht wird,so dass die Batterie erst unmittelbar vor der Benutzung des Messgeräts gebildet wird und so Probleme aufgrund einer Entladung der Batterie im Zeitraum zwischen der Herstellung des Messgeräts und seiner Benutzung vermieden werden. Da der Messvorgang innerhalb einer definierten Zeit stattfindet, enthält die Stromversorgungseinrichtung vorzugsweise eine aufladbare Speicherkapazität, die die für die Zeit der Messung benötigte Energie abrufbar bereitstellt. Die Speicherkapazität, z. B. ein Doppelschichtkondensator (Super-Cap oder Super Capacitor), kann beispielsweise von der Solarzelle oder der Batterie direkt oder über einen Spannungswandler auf eine definierte Spannung aufgeladen werden. Ist der gewünschte Ladezustand erreicht, so kann die Messung automatisch oder auf Anforderung durch den Benutzer, ggf. nach vorheriger Anzeige des Ladezustandes über die Anzeigevorrichtung, ausgelöst werden. Die Aufladung der Speicherkapazität kann auch von außen kontaktgebunden oder kontaktlos, z. B. über induktive oder kapazitive Kopplung, erfolgen, so dass dann auf die Solarzelle oder Batterie verzichtet werden kann.

In Verbindung mit der Bestimmung ausgewählter Analyten im Blut, beispielsweise Glucose oder Lactat, ist in dem Messgerät vorzugsweise eine Stechhilfe, insbesondere eine Nadel oder einen Dorn, zur Gewinnung der Blutprobe integriert. Die Stechhilfe kann dabei außerhalb des Testfeldes oder der Testfelder angeordnet sein, so dass nach dem Stechen der Bluttropfen auf das Testfeld gebracht werden muss. Alternativ ist die Stechhilfe im oder unmittelbar am Testfeld angeordnet, so dass mit dem Stechen der Bluttropfen unmittelbar auf das Testfeld gelangt.

Um eine Mehrfachbenutzung des erfindungsgemäßen Messgeräts zu ermöglichen, ist gemäss Anspruch 1 mindestens ein weiteres Testelement mit Testfeld und Detektor vorgesehen, wobei die Detektoren über eine Auswahlschaltung an der Auswerteschaltung angeschlossen sind. Die Auswahlschaltung verbindet immer nur einen der Detektoren und diesen nur zur einmaligen Benutzung mit der Auswerteschaltung und wählt nach erfolgter Messung einen noch nicht benutzten Biosensor aus. Durch die mehrfache Benutzbarkeit kann das erfindungsgemäße Messgerät weitaus kostengünstiger und für den Benutzer bequemer sein, als ein nur einmalig benutzbares Gerät. Dabei stellt die Auswahlschaltung, die Bestandteil der Steuereinrichtung sein kann, sicher, dass immer nur ein einziges und noch nicht benutztes Testelement mit seinem Detektor an der Auswerteschaltung angeschlossen ist.

Durch geeignete Ansteuerung der Anzeigevorrichtung kann dem Benutzer das dem jeweils aktuell benutzbaren Testelement zugeordnete Testfeld und im Weiteren nach Benutzung aller vorhandener Testelemente die Unbrauchbarkeit des Messgeräts angezeigt werden. Um die unbenutzten Testfelder zu schützen, können diese mit einer vor der Benutzung abzunehmenden Abdeckung, insbesondere einer Folie abgedeckt sein, wobei bereits die nicht mehr vorhandene Abdeckung dem Benutzer anzeigt, welche Testfelder nicht mehr benutzbar sind. Die Auswahlschaltung kann über Detektionsmittel, z. B. eine unterbrechbare elektrische Leiterschleife, die abgezogenen Abdeckungen detektieren, um so bereits benutzte, aber auch versehentlich oder aufgrund von Beschädigungen der Abdeckungen freigelegte Testfelder von der weiteren Benutzung auszuschließen. Das kann in der Weise erfolgen, dass die Auswahlschaltung dann, wenn sie erstmals das Abziehen einer Folie detektiert, den zugeordneten Detektor - falls dieser noch nicht ausgewählt worden ist - auswählt und nach einer vorgegebenen, für die Messung ausreichenden Zeitdauer von jeder weiteren Benutzung ausschließt. Alternativ oder ergänzend kann die Auswahlschaltung Mittel aufweisen, die auf die Betätigung eines Bedienelementes am Messgerät, z. B. eines Bedienknopfes oder auf das Aufbringen der Flüssigkeitsprobe auf eines der Testfelder ansprechen und den zugeordneten Detektor - falls dieser noch nicht ausgewählt worden ist - für die Messung auswählen.

Gemäss Anspruch 1 sind die Testfelder im Randbereich des flachen Trägers in durch Bruchlinien voneinander getrennten Zonen angeordnet, wobei jede Zone nach Benutzung des darin liegenden Testfeldes von dem Träger abbrechbar ist. Dabei sind die Testfelder in den Zonen vorzugsweise derart angeordnet, dass sie von jeweils einer benachbarten Zone abgedeckt sind und erst durch Abbrechen dieser benachbarten Zone zugänglich gemacht werden. Dadurch wird erreicht, dass die Testfelder nur in einer vorgegebenen Reihenfolge nacheinander benutzbar sind. Auch hier kann die Auswahlschaltung über Detektionsmittel die abgebrochenen Zonen detektieren und so die noch benutzbaren Testfelder erkennen.

Zur weiteren Erläuterung der Erfindung wird im Folgenden auf die Figuren der Zeichnung Bezug genommen; im Einzelnen zeigen
- Figur 1: ein erstes Ausführungsbeispiel des erfin- dungsgemäßen Messgeräts mit einem Testele- ment als vereinfachtes Blockschaltbild,
- Figur 2: eine Modifikation des Ausführungsbeispiels nach Figur 1,
- Figur 3: ein vereinfachtes Beispiel für die elektri- sche Schaltung des erfindungsgemäßen Messge- rätes,
- Figur 4: ein weiteres Ausführungsbeispiel des erfin- dungsgemäßen Messgeräts mit mehreren Test- elementen als vereinfachtes Blockschaltbild und die
- Figuren 5 bis 8: Ansichten unterschiedlicher Ausführungsbei- spiele des erfindungsgemäßen Messgeräts.

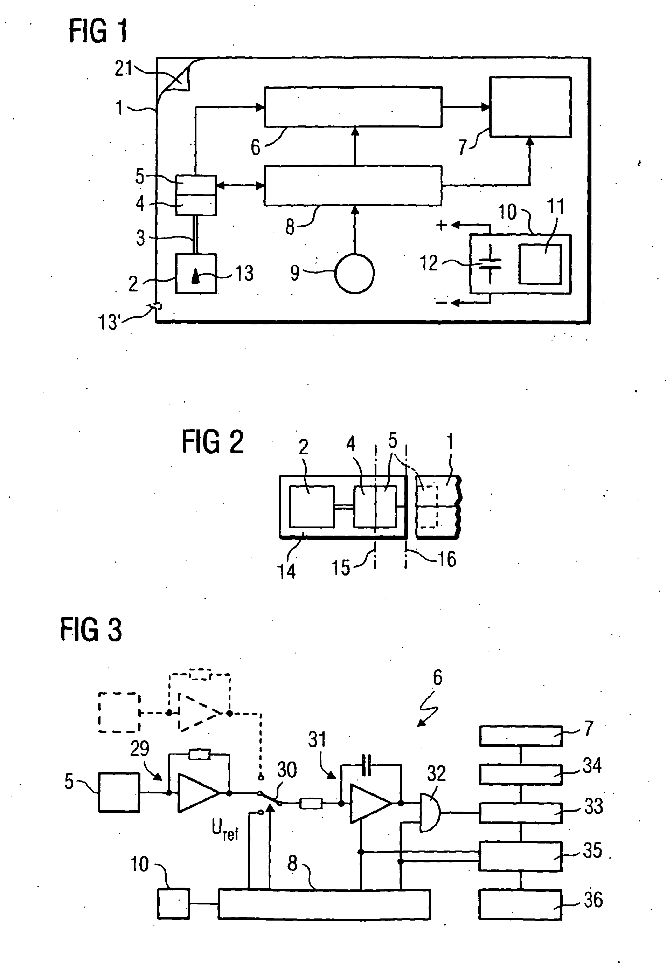

Figur 1 zeigt einen scheckkartenförmigen Träger 1 auf dem ein Testfeld 2 zur Aufbringung einer Flüssigkeitsprobe ausgebildet sind. Das Testfeld 2 kommuniziert über einen Flüssigkeitspfad 3 oder unmittelbar direkt mit einem Testelement 4, das durch den zu bestimmenden Analyten eine detektierbare Veränderung erfährt. Diese Veränderung wird mittels eines Detektors 5 beispielsweise optisch oder elektrochemisch detektiert, der in Abhängigkeit davon ein elektrisches Detektorsignal erzeugt. Weist das Testelement 4 beispielsweise eine auf den zu bestimmenden Analyten ansprechende biologische oder chemische Komponente, z. B. ein Enzym, einen Antikörper oder einen Mikroorganismus auf, so bilden das Testelement 4 und der Detektor 5 zusammen einen sogenannten Biosensor. Beispiele für solche Sensoraufbauten sind aus der US 5 997 817 oder der US 6 036 919 bekannt. Soll mehr als ein Analyt aus der Flüssigkeitsprobe bestimmt werden, so können natürlich zusätzliche Testelemente mit Detektoren vorgesehen werden, die mit dem Testfeld 2 kommunizieren.

Der Detektor 5 ist an einer Auswerteschaltung 6 angeschlossen, die das Detektorsignal zu einem Messergebnis auswertet und dieses mittels einer Anzeigevorrichtung 7 visualisiert. Die Steuerung des Messvorganges erfolgt durch eine Steuereinrichtung 8, die dazu an dem Detektor 5, der Auswerteschaltung 6 und der Anzeigevorrichtung 7 angeschlossen ist. Mittels eines ebenfalls mit der Steuereinrichtung 8 verbundenen Bedienelements 9, im einfachsten Fall ein einziger Bedienknopf, können Basisfunktionen des Messgeräts, wie z. B. Ein- und Ausschalten, Reset, Betätigung einer auf der Anzeigevorrichtung 7 wiedergegebenen Anweisung usw., ausgeübt werden. Die Anzeigevorrichtung 7 kann dem Benutzer die vorzunehmenden Bedienhandlungen in Form eines Textes oder Piktogramms anzeigen sowie den Funktionsstatus des Messgeräts mitteilen. Die Stromversorgung der elektrischen Gerätekomponenten 5 bis 9 erfolgt durch eine Stromversorgungseinrichtung 10, vorzugsweise mit einer Solarzelle 11, die z. B. aus Piatzgründen unter der Anzeigevorrichtung 7 angeordnet und von dem durch sie hindurchtretenden Umgebungslicht beleuchtet werden kann. Die Stromversorgungseinrichtung 10 enthält im Weiteren vorzugsweise eine aus der Solarzelle 11 auf eine definierte Spannung aufladbare Speicherkapazität 12, die die für den Messvorgang benötigte Energie schnell und in ausreichender Höhe abrufbar bereitstellt. Die elektrischen Gerätekomponenten 5 bis 12 sind alle oder zumindest aber teilweise, beispielsweise mit Ausnahme der Stromversorgungseinrichtung 10 und/oder der Anzeigevorrichtung 7, in Polymerelektronik ausgebildet und z. B. auf dem Plastikträger 1 aufgedruckt.

Der Träger 1 mit den darauf ausgebildeten Gerätekomponenten ist mit einer Abdeckung 21, hier in Form einer abziehbaren Folie, abgedeckt, die die Gerätekomponenten 2 bis 12 gegen Umwelteinflüsse von außen schützt. Die Abdeckung 21 kann lichtundurchlässig sein, was zum einen einen Lichtschutz bewirkt und zum anderen das Detektieren des Abziehens der Folie 21 vor dem Gebrauch des Messgerätes durch die Solarzelle 11 oder ein anderes, hier nicht gezeigtes, elektrooptisches Detektorelement ermöglicht. Ist die Abdeckung 21 als Dauerschutz auch während des Gebrauchs der Messgerätes vorgesehen, so ist sie zumindest im Bereich der Anzeigevorrichtung 7 und der Solarzelle 11 transparent und weist im Bereich des Testfeldes 2 eine Öffnung auf.

Im Bereich des Testfeldes 2 ist auf dem Träger 1 eine Stechhilfe, hier ein Dorn 13, zur Gewinnung von Blut als Flüssigkeitsprobe integriert. Der Dorn 13 kann beispielsweise unter einer blasenförmigen Abdeckung über dem Testfeld 2 angeordnet sein, die auf Fingerdruck entgegen einer mechanischen Vorspannung nachgibt, so dass der Dorn 13 in den Finger sticht. Wie gestrichelt angedeutet ist, kann die Stechhilfe 13', die aus dem Material des kartenförmigen Trägers 1 bestehen kann, alternativ auch in der Nähe zum Testfeld 2 angeordnet sein.

Wie Figur 2 zeigt, kann das Testfeld 2 mit dem Testelement 4 und ggf. dem Detektor 5 auf einem separaten Träger 14 ausgebildet sein, der über eine Schnittstelle 15 bzw. 16 an dem kartenförmigen Träger 1 ankoppelbar ist.

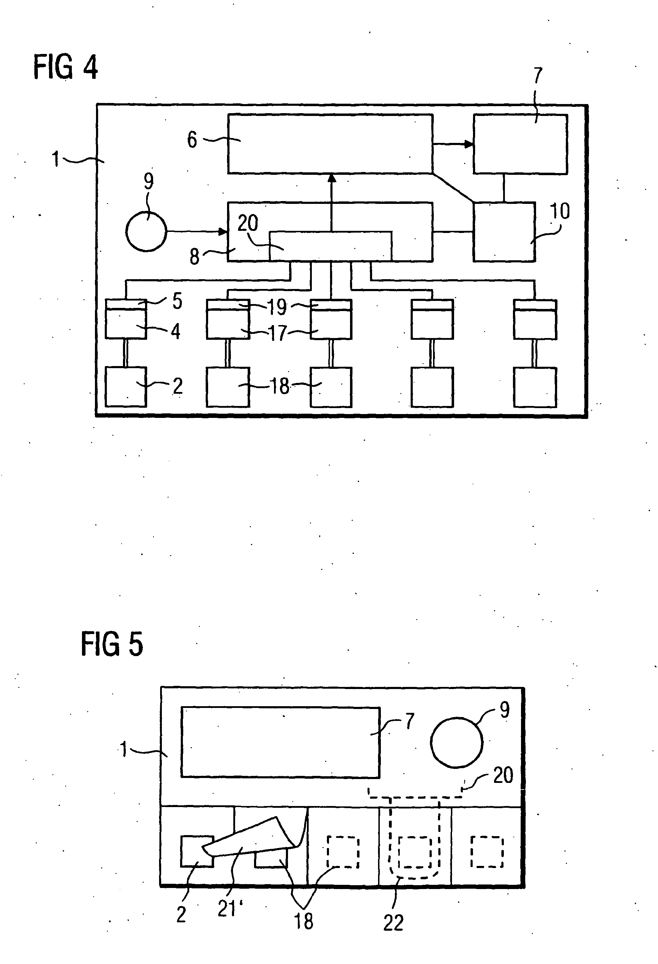

Figur 3 zeigt ein Beispiel für die elektrische Schaltung des erfindungsgemäßen Messgerätes. Die Auswerteschaltung 6 weist eingangsseitig einen Verstärker 29 auf, der je nach Art des Detektors 5 und des von diesem erzeugten Detektorsignals dessen Ausgangsspannung verstärkt oder den Ausgangsstrom in eine Spannung umsetzt. Die so erhaltene Spannung wird über einen steuerbaren Schalter 30 einem Analog-/Digital-Umsetzer (ADU) 31, hier einem Dual-Slope-ADU, zugeführt, der ein digitales serielles Messergebnis erzeugt. Dieses Messergebnis wird über ein Gatter 32 taktgesteuert in ein Schieberegister 33 eingelesen, von wo aus es über eine Decodierlogik 34 der Anzeigevorrichtung 7 zugeführt und dort visualisiert wird. Über den steuerbaren Schalter 30 kann der Eingang des Analog-/Digital-Umsetzers 31 an eine Referenzspannung U_{ref} geschaltet werden, die hier von der Steuereinrichtung 8 bereitgestellt wird. Ferner kann der Schalter 30, wie gestrichelt angedeutet ist, zur Auswahl weiterer Detektoren dienen, so wie dies später in Verbindung mit Figur 4 erläutert wird.. Die Steuereinrichtung 8 steuert dazu den Schalter 30 an und erzeugt auch ein Rücksetzsignal für den Analog-/Digital-Umsetzer 31 und das Taktsignal für das Gatter 32:

Schließlich steuert die Steuereinrichtung 8 auch die Kalibration des Messgerätes, indem das in dem Schieberegister 33 enthaltene digitale Messergebnis mittels einer Recheneinrichtung 35 aufgrund von in einem Kalibrationsdatenspeicher 36 enthaltenen Kalibrationsdaten korrigiert wird. Bei dem Kalibrationsdatenspeicher 36 kann es sich um einen programmierbaren Speicher (z. B. EEPROM) handeln, in den die Kalibrationsdaten kontaktgebunden oder beispielsweise mittels eines Transponders kontaktlos einlesbar sind, oder um eine elektrothermisch, mittels Laser oder auf sonstige Weise änderbare, elektrische Verbindungsstruktur. Aufgrund nicht vollständig kontrollierbarer Herstellungsbedingungen ist es nämlich notwendig, eine chargenspezifische Kalibration der Messgeräte vorzunehmen. Dies erfolgt im Regelfall dadurch, dass Messgeräte nach Fertigstellung anhand von Referenzproben bekannter Konzentration getestet werden und die Wiederfindung der bekannten Konzentration in dem Messergebnis geprüft wird. Aufgrund von chargenspezifischen Schwankungen kann es dann notwenig sein, dem Messgerät Kalibrationsdaten zu übermitteln, mit denen die Messergebnisse korrigiert werden. Es ist natürlich möglich, die Kalibration auch schon während der Herstellung der Messgeräte einzubringen, indem einzelne Messgeräte nach ihrer Fertigstellung kalibriert werden, und die Kalibrierdaten in die übrigen Messgeräte der Charge während ihrer Herstellung eingebracht werden.

Bei dem in Figur 4 gezeigten Ausführungsbeispiel sind auf dem kartenförmigen Träger 1 neben dem Testelement 4 mit dem Testfeld 2 und dem Detektor 5 weitere Testelemente 17 mit Testfeldern 18 und Detektoren 19 ausgebildet. Die Detektoren 19 sind an einer Auswahlschaltung 20 angeschlossen, die Bestandteil der Steuereinrichtung 8 ist und immer nur ein und zwar noch unbenutztes Testelement auswählt, den zugehörigen Detektor mit der Auswerteschaltung 6 verbindet und nach erfolgter Messung ein bisher noch nicht benutztes Testelement für die nächste Messung auswählt. Die Auswerteschaltung 6 wertet das Detektorsignal des jeweils mit ihr verbundenen Detektor zu einem Messergebnis aus, das mittels der Anzeigevorrichtung 7 visualisiert wird. Mittels des Bedienelements 9 können Basisfunktionen des Messgeräts ausgeübt werden. Die Stromversorgung der elektrischen Gerätekomponenten 5 bis 9, 19 und 20 erfolgt durch die Stromversorgungseinrichtung 10. Auch hier sind die elektrischen Gerätekomponenten 5 bis 10, 19 und 20 zumindest teilweise in Polymerelektronik ausgebildet und z. B. auf dem Plastikträger 1 aufgedruckt.

Figur 5 zeigt die Ansicht eines Ausführungsbeispiels des erfindungsgemäßen Messgeräts, wobei der Träger 1 mit den Testfeldern 2, 18, der Anzeigevorrichtung 7 und dem Bedienelement 9 sichtbar sind. Die Testfelder 2, 18 sind jeweils durch abziehbare Abdeckungen 21, hier in Form von Folien, abgedeckt. Wie am Beispiel der einem der weiteren Testfelder 18 zugeordneten Folie 21' zu sehen ist, sind Detektionsmittel 22 in Form einer unterbrechbaren Leitungsschleife o. ä. vorgesehen, dies der Auswahlschaltung 20 signalisieren, sobald eine Folie entfernt oder beschädigt wurde. Durch das Entfernen einer Folie 21' kann das darunter liegende Testfeld mit dem zugeordneten Testelement und Detektor für eine vorgegebene Zeitdauer für die Messung ausgewählt werden. Nach Ablauf dieser Zeitdauer und/oder Beendigung der Messung ist das betreffende Testelement für eine künftige Auswahl bzw. Benutzung gesperrt.

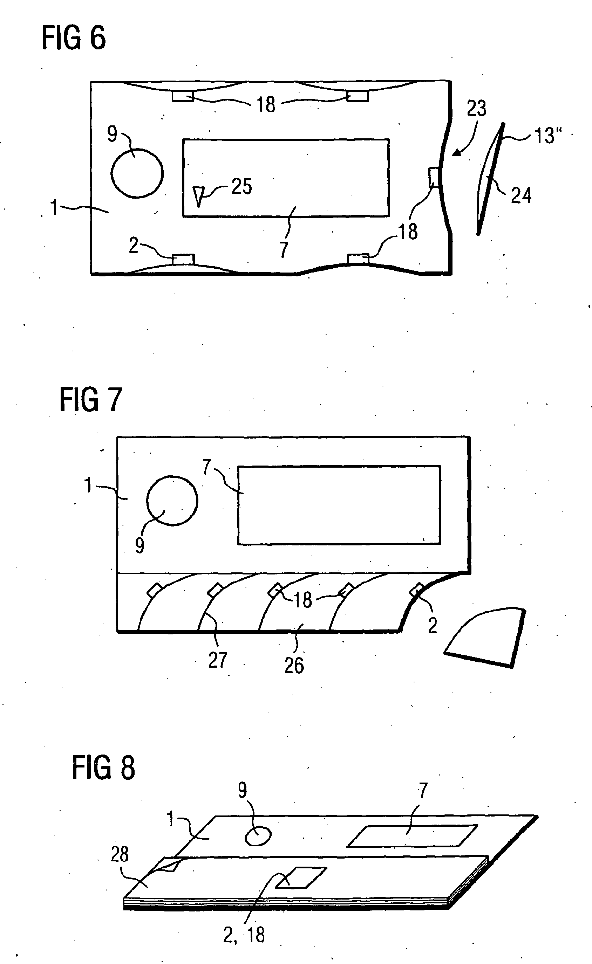

Bei dem in Figur 6 gezeigten Ausführungsbeispiel sind die Testfelder 2, 18, so wie z. B. aus der.US 5 997 817 bekannt, ausgebildet und am Rand des kartenförmigen Trägers 1 in muldenartigen Aussparungen 23 angeordnet und dort durch abbrechbare Abdeckungen 24 geschützt. Für den Benutzer ist sofort sichtbar, welche der Testfelder 2, 18 bereits benutzt worden sind. Das Abbrechen der Abdeckungen 24 kann ähnlich wie das Abziehen der Folien 21' in dem Beispiel nach Figur 5 detektiert werden. Die Anzeigevorrichtung 7 zeigt, hier durch einen Pfeil 25, das für die nächste Benutzung ausgewählte Testfeld, z. B. 2, an. Die Abdeckungen 24 können, wie hier gezeigt, scharfkantig ausgebildet sein, so dass sie nach dem Abbrechen als Stechhilfe 13" zur Blutgewinnung dienen können.

Bei dem Ausführungsbeispiel nach Figur 7 sind die Testfelder 2, 18 am Rand des kartenförmigen Trägers 1 in Zonen 26 angeordnet, die durch Bruchlinien 27 voneinander getrennt sind, so dass die einzelnen Zonen 26 nach Benutzung der darin enthaltenen Testfelder, Testelemente und ggf. Detektoren von dem Träger 1 abgebrochen und so nicht ein weiteres Mal benutzt werden können. Damit dies auch wirklich geschieht, sind die Testfelder 2, 18 in den Bereichen der Bruchlinien 27 zu jeweils einer benachbarten Zone 26 angeordnet, so dass die Testfelder 2, 18 erst durch das Abbrechen dieser benachbarten Zone zugänglich gemacht werden.

Bei dem in Figur 8 gezeigten Ausführungsbeispiel sind die Testfelder 2, 18 mit den hier nicht sichtbaren Testelementen und Detektoren auf separaten Trägerfolien 28 ausgebildet, die übereinander liegend auf dem Träger 1 mit den übrigen Gerätekomponenten, z. B. 7 und 9, aufgebracht sind. Die Trägerfolien 28 sind nacheinander abziehbar, wobei nur das Testfeld auf der jeweils obersten Trägerfolie 28 benutzbar ist und die darunter liegenden Testfelder geschützt sind. Zur elektrischen Verbindung der Detektoren mit der Auswahlschaltung auf dem Träger 1 sind die Trägerfolien 28 zu dem Träger 1 hin durchkontaktiert. Das Abziehen von Trägerfolien 28 kann, wie bereits oben erläutert, elektrisch oder optisch detektiert werden. Wie gestrichelt angedeutet ist, können sich die Trägerfolien 28 über die gesamte Fläche des Trägers 1 erstrecken und sind dann im Bereich der Anzeigevorrichtung 7 und des Bedienknopfes 9 transparent.

## Patentansprüche

1. Messgerät zur Bestimmung eines Analyten in einer Flüssigkeitsprobe, mit folgenden Gerätekomponenten: ein Testelement (4) mit einem Testfeld (2) zur Aufbringung der Flüssigkeitsprobe, wobei das Testelement (4) durch den zu bestimmenden Analyten eine detektierbare Veränderung erfährt, ein Detektor (5), der die Veränderung detektiert und in Abhängigkeit davon ein elektrisches Detektorsignal erzeugt, eine dem Detektor (5) nachgeordnete Auswerteschaltung (6), die das Detektorsignal zu einem Messergebnis auswertet, eine an der Auswerteschaltung (6) angeschlossene Anzeigevorrichtung (7) zur Anzeige des Messergebnisses und eine Stromversorgungseinrichtung (10) zur Stromversorgung der elektrischen Gerätekomponenten (5 bis 8), **dadurch gekennzeichnet, dass** die elektrischen Gerätekomponenten (5 bis 10) zumindest teilweise auf der Basis von Polymerelektronik ausgebildet sind,
dass mindestens ein weiteres Testelement (17) mit Testfeld (18)und Detektor (19) vorgesehen ist,
dass die Detektoren (5, 19) über eine Auswahlschaltung (20) an der Auswerteschaltung (6) angeschlossen sind und dass die Auswahlschaltung (20) immer nur einen der Detektoren (5, 19) und diesen nur zur einmaligen Benutzung mit der Auswerteschaltung (20) verbindet und nach erfolgter Messung einen noch nicht benutzten Detektor auswählt,
wobei
bei Ausbildung der Gerätekomponenten auf einem flachen Träger (1) die Testfelder (2, 18) im Randbereich des Trägers (1) in durch Bruchlinien (27) voneinander getrennten Zonen (26) angeordnet sind und dass jede Zone (26) nach Benutzung des darin liegenden Testfeldes von dem Träger (1) abbrechbar ist.

2. Messgerät nach Anspruch 1, **dadurch gekennzeichnet, dass** das Testfeld (2) und das Testelement (4) oder das Testfeld (2), das Testelement (4) und der Detektor (5) in einer ersten Geräteeinheit (14) ausgebildet sind, die über eine Schnittstelle (15, 16) an einer die übrigen Gerätekomponenten (6 bis 10) enthaltenden weiteren Geräteeinheit (Träger 1) ankoppelbar ist.

3. Messgerät nach einem der vorangehenden Ansprüche, **dadurch gekennzeichnet, dass** die Gerätekomponenten weiterhin eine die Bestimmung des Analyten in einem Messvorgang steuernde Steuereinrichtung (8) umfassen.

4. Messgerät nach Anspruch 3, **dadurch gekennzeichnet, dass** die Steuereinrichtung (8) Mittel zum Aktivieren des Messvorganges aufweist, die auf mindestens eines der folgenden Ereignisse ansprechen: ein Bedienelement (9) an dem Messgerät wird betätigt, eine Abdeckung an dem Messgerät wird entfernt, das Testelement (4) wird an den kartenförmigen Träger (1) angekoppelt, die Flüssigkeitsprobe wird auf das Testfeld (2) aufgebracht.

5. Messgerät nach Anspruch 3 oder 4, **dadurch gekennzeichnet, dass** die Steuereinrichtung (8) Mittel zum Verhindern weiterer Messvorgänge mit ein und demselben Testelement (4) aufweist.

6. Messgerät nach Anspruch 5, **dadurch gekennzeichnet, dass** Anzeigemittel, insbesondere die Anzeigevorrichtung (7), vorhanden sind, die durch die Steuereinrichtung (8) zur Anzeige der Unbrauchbarkeit des Messgerätes ansteuerbar sind.

7. Messgerät nach einem der vorangehenden Ansprüche, **dadurch gekennzeichnet, dass** die Auswerteschaltung (6) einen Kalibrationsdatenspeicher (36) mit Kalibrationsdaten zur Korrektur der anzuzeigenden Messergebnisse aufweist.,

8. Messgerät nach einem der vorangehenden Ansprüche, **dadurch gekennzeichnet, dass** das Messgerät eine zumindest im Bereich des Testfeldes (2) entfernbare und gegenüber Umgebungseinflüssen, insbesondere Wasserdampf und/oder Sauerstoff, abdichtende Abdeckung (21), insbesondere Verpackung, aufweist.

9. Messgerät nach einem der vorangehenden Ansprüche, **dadurch gekennzeichnet, dass** die Stromversorgungseinrichtung (10) eine aufladbare Speicherkapazität (12) aufweist.

10. Messgerät nach einem der vorangehenden Ansprüche, **dadurch gekennzeichnet, dass** in ihm eine Stechhilfe (13, 13', 13"), insbesondere eine Nadel oder einen Dorn, zur Gewinnung von Blut als Flüssigkeitsprobe integriert ist.

11. Messgerät nach Anspruch 10, **dadurch gekennzeichnet, dass** die Auswahlschaltung (20) Anzeigemittel, insbesondere die Anzeigevorrichtung (7), in der Weise ansteuert, dass diese das dem aktuell benutzbaren Detektor zugeordnete Testfeld anzeigt.

12. Messgerät nach Anspruch 11, **dadurch gekennzeichnet, dass** die Testfelder (2, 18) in den Zonen (26) derart angeordnet sind, dass sie jeweils von einer benachbarten Zone abgedeckt und erst nach Abbrechen dieser benachbarten Zone zugänglich sind.

13. Messgerät nach Anspruch 11 oder 12, **dadurch gekennzeichnet, dass** in den Bereichen der Bruchlinien (27) mit der Auswahlschaltung (20) verbundene Detektionsmittel angeordnet sind und dass die Auswahlschaltung (20) abgebrochene Zonen detektiert.

## Claims

1. Measuring apparatus for determining an analyte in a liquid sample, having the following apparatus components: a test element (4) with a test field (2) for applying the liquid sample, the test element (4) experiencing a detectable modification due to the analyte to be determined, a detector (5) which detects the modification and generates an electrical detector signal as a function of it, an evaluation circuit (6) arranged downstream of the detector (5), which evaluates the detector signal to form a measurement result, a display device (7) connected to the evaluation circuit (6) for displaying the measurement result, and an electrical power supply (10) for supplying electrical power to the electrical apparatus components (5 to 8), **characterized in that** at least some of the electrical apparatus components (5 to 10) are formed on the basis of polymer electronics, **in that** at least one further test element (17) with a test field (18) and a detector (19) is provided, **in that** the detectors (5, 19) are connected via a selection circuit (20) to the evaluation circuit (6) and **in that** the selection circuit (20) always selects only one of the detectors (5, 19) and joins it only for single use to the selection circuit (20) and subsequently selects an as yet unused detector after the measurement has taken place, wherein, when the apparatus components are formed on a flat support (1), the test fields (2, 18) are arranged in the edge region of the support (1) in zones (26) separated from one another by break lines (27), and **in that** each zone (26) can be broken off from the support (1) after the test field lying in it has been used.

2. Measuring apparatus according to Claim 1, **characterized in that** the test field (2) and the test element (4) or the test field (2), the test element (4) and the detector (5) are formed in a first apparatus unit (14), which can be coupled via an interface (15, 16) to a further apparatus unit (support 1) which contains the other apparatus components (6 to 10).

3. Measuring apparatus according to one of the preceding claims, **characterized in that** the apparatus components furthermore comprise a controller (8) which controls the determination of the analyte in a measurement process.

4. Measuring apparatus according to Claim 3, **characterized in that** the controller (8) has means for activating the measurement process, which respond to at least one of the following events: an operating element (9) on the measuring apparatus is actuated, a cover on the measuring apparatus is removed, the test element (4) is coupled to the card-shaped support (1), the liquid sample is applied onto the test field (2).

5. Measuring apparatus according to Claim 3 or 4, **characterized in that** the controller (8) has means for preventing further measurement processes with the same test element (4).

6. Measuring apparatus according to Claim 5, **characterized in that** there are display means, in particular the display device (7), which can be driven by the controller (8) in order to display that the measuring apparatus is unusable.

7. Measuring apparatus according to one of the preceding claims, **characterized in that** the evaluation circuit (6) has a calibration data memory (36) with calibration data for correcting the measurement results to be displayed.

8. Measuring apparatus according to one of the preceding claims, **characterized in that** the measuring apparatus has a removable cover (21), in particular packaging, at least in the region of the test field (2), which seals against environmental effects, in particular water vapor and/or oxygen.

9. Measuring apparatus according to one of the preceding claims, **characterized in that** the electrical power supply (10) has a chargeable storage capacitor (12).

10. Measuring apparatus according to one of the preceding claims, **characterized in that** a pricking aid (13, 13', 13"), in particular a needle or a spike, is integrated in it in order to obtain blood as the liquid sample.

11. Measuring apparatus according to Claim 10, **characterized in that** the selection circuit (20) drives display means, in particular the display device (7), so that it displays the test field assigned to the currently usable detector.

12. Measuring apparatus according to Claim 11, **characterized in that** the test fields (2, 18) in the zones (26) are arranged so that they are respectively covered by a neighboring zone and are accessible only after this neighboring zone has been broken off.

13. Measuring apparatus according to Claim 11 or 12, **characterized in that** detection means joined to the selection circuit (20) are arranged in the regions of the break lines (27) and **in that** the selection circuit (20) detects zones having been broken off.

## Revendications

1. Appareil de mesure pour déterminer un analyte dans un échantillon liquide comportant les composants suivants : un élément de test (4) avec une zone de test (2) pour déposer l'échantillon liquide, où l'élément de test (4) subit une modification détectable du fait de l'analyte à déterminer, un détecteur (5), qui détecte la modification et qui produit un signal électrique de détecteur en fonction de celle-ci, un circuit d'évaluation (6) placé en aval du détecteur (5), qui évalue le signal du détecteur pour donner un résultat de mesure, un dispositif d'affichage (7) raccordé au circuit d'évaluation (6) pour l'affichage du résultat de mesure et un système d'alimentation électrique (10) pour alimenter en électricité les composants électriques de l'appareil (5 à 8), **caractérisé en ce que** les composants électriques de l'appareil (5 à 10) sont au moins partiellement formés à base de composants électroniques polymères,
**en ce qu'**au moins un autre élément de test (17) est doté d'une zone de test (18) et d'un détecteur (19), **en ce que** les détecteurs (5, 19) sont raccordés au circuit d'évaluation (6) par le biais d'un circuit de sélection (20) et **en ce que** le circuit de sélection (20) ne relie toujours qu'un des détecteurs (5, 19) et ne relie celui-ci que pour une unique utilisation avec le circuit d'évaluation (6) et sélectionne, une fois la mesure réalisée, un détecteur qui n'a pas été encore utilisé,
dans lequel
lors de la formation des composants de l'appareil sur un support plat (1), les zones de test (2, 18) sont disposées à la périphérie du support (1) dans des zones (26) séparées les unes des autres par des lignes de rupture (27), et **en ce que** chaque zone (26) peut être détachée du support (1) après utilisation de la zone de test qui est située dessus.

2. Appareil de mesure selon la revendication 1, **caractérisé en ce que** la zone de test (2) et l'élément de test (4) ou la zone de test (2), l'élément de test (4) et le détecteur (5) sont formés dans une première unité de l'appareil (14), qui peut être couplée par le biais d'une interface (15, 16) à une autre unité de l'appareil (support 1) comprenant les composants restants de l'appareil (6 à 10).

3. Appareil de mesure selon l'une des revendications précédentes, **caractérisé en ce que** les composants de l'appareil comprennent également un système de commande (8) qui commande la détermination de l'analyte dans une opération de mesure.

4. Appareil de mesure selon la revendication 3, **caractérisé en ce que** le système de commande (8) présente des moyens pour l'activation de l'opération de mesure, qui répondent au moins à l'un des évènements suivants : un élément de commande (9) sur l'appareil de mesure est actionné, un recouvrement de l'appareil de mesure est retiré, l'élément de test (4) est couplé au support (1) en forme de carte, l'échantillon liquide est déposé sur la zone de test (2).

5. Appareil de mesure selon la revendication 3 ou 4, **caractérisé en ce que** le système de commande (8) présente des moyens pour empêcher d'autres opérations de mesure avec un et même élément de test (4).

6. Appareil de mesure selon la revendication 5, **caractérisé en ce qu'**il existe des moyens d'affichage, en particulier le dispositif d'affichage (7), qui peuvent être commandés par le système de commande (8) pour afficher l'inutilisabilité de l'appareil de mesure.

7. Appareil de mesure selon l'une des revendications précédentes, **caractérisé en ce que** le circuit d'évaluation (6) présente une mémoire de calibration (36) avec des données de calibration pour corriger les résultats de mesure à afficher.

8. Appareil de mesure selon l'une des revendications précédentes, **caractérisé en ce que** l'appareil de mesure présente un recouvrement (21), en particulier un emballage, étanche vis-à-vis des influences extérieures, en particulier de la vapeur d'eau et/ou de l'oxygène, et amovible au moins au niveau de la zone de test (2).

9. Appareil de mesure selon l'une des revendications précédentes, **caractérisé en ce que** le système d'alimentation électrique (10) présente une capacité de stockage rechargeable (12).

10. Appareil de mesure selon l'une des revendications précédentes, **caractérisé en ce qu'**un accessoire de piqûre (13, 13', 13"), en particulier une aiguille ou une seringue, est intégré pour le prélèvement du sang sous la forme d'un échantillon liquide.

11. Appareil de mesure selon la revendication 10, **caractérisé en ce que** le circuit de sélection (20), commande les moyens d'affichage, en particulier le dispositif d'affichage (7), de manière à ce que celui-ci affiche la zone de test affectée au détecteur actuellement utilisable.

12. Appareil de mesure selon la revendication 11, **caractérisé en ce que** les zones de test (2, 18) sont disposées dans les zones (26) de manière à être recouvertes chacune par une zone voisine et accessibles seulement après détachement de cette zone voisine.

13. Appareil de mesure selon la revendication 11 ou 12, **caractérisé en ce que** des moyens de détection reliés au circuit de sélection (20) sont disposés aux endroits des lignes de rupture (27) et **en ce que** le circuit de sélection (20) détecte les zones détachées.
